# EUROPEAN PATENT APPLICATION

(11) **EP 3 792 271 A1**
(43) Date of publication of application: **17.03.2021**
(21) Application number: 20195789.1
(22) Date of filing: 11.09.2020
(51) Int. Cl.: C07H 1/00, C07H 19/073

(54) **A PROCESS FOR THE PREPARATION OF BRIVUDINE**

(30) Priority: 13.09.2019 IN 201941036877
(71) Applicant: Aurobindo Pharma Limited, Hyderabad 500084 (IN)
(72) Inventor: Nandi, Sukumar, 500 084 Hyderabad (IN); Sharma, Mukul, 500 084 Hyderabad (IN); Dwarakapally, Sreedhar Reddy, 500 084 Hyderabad (IN); Miriyala, Ravikumar Gupta, 500 084 Hyderabad (IN); Penupothula, Veera Vijay Kumar, 500 084 Hyderabad (IN); Meenakshisunderam, Sivakumaran, 500 084 Hyderabad (IN)
(74) Representative: Hamm&Wittkopp Patentanwälte PartmbB

(57) **Abstract**

The present invention relates to a novel compound 2'-deoxyuridine of Formula (II) and its conversion to Brivudine of Formula (I) or pharmaceutically acceptable salts thereof.

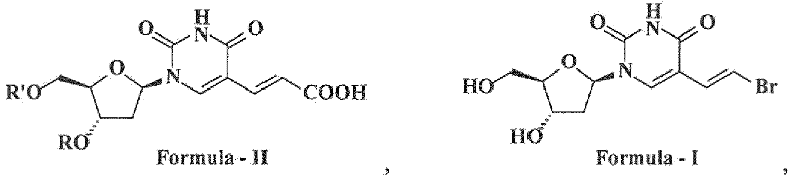

Wherein, R and R' are same or different selected from hydroxy protecting groups.

## Description

### FIELD OF THE INVENTION

The present invention relates to a novel compound 2'-deoxyuridine of Formula (II) and its conversion to Brivudine of Formula (I) or pharmaceutically acceptable salts thereof. 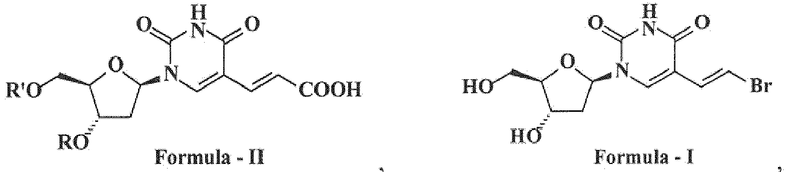 wherein, R and R' are same or different selected from hydroxy protecting groups.

### BACKGROUND OF THE INVENTION

Brivudine is chemically known as 5-[(lE)-2-bromoethenyl]-2'-deoxyuridine. Brivudine is an analogue of the nucleoside thymidine with high and selective antiviral activity against varicella zoster virus and herpes simplex virus. Brivudine is an antiviral drug approved under the brand name Zostex® for treatment of herpes zoster. Brivudine is also useful to inhibit the upregulation of chemoresistance genes (Mdr1 and DHFR) during chemotherapy. Overall, the gene expression changes associated with Brivudine treatment result in the decrease or prevention of chemoresistance. In addition, it has been shown to enhance the cytolytic activity of NK-92 natural killer cells towards a pancreatic cancer cell line in vitro.

Brivudine (I) is disclosed first time in DE 2915254. This patent discloses a process for the preparation of Brivudine by coupling E-5-(2-bromovinyl) uracil with 1-chloro-2-deoxy-3,5-di-O-p-toluoyl-α-D-erythro-pentofuranose to obtain E-5-(2-bromovinyl)-3',5'-di-O-p-toluoyl-2'-deoxyuridine as a mixture of α and β isomers. This mixture was subjected to chromatographic purification to obtain pure β-isomer. In the subsequent stage E-5-(2-bromovinyl)-3',5'-di-O-p-toluoyl-2'-deoxyuridine was treated with sodium methoxide to yield Brivudine. The process is depicted in the below as Scheme I:

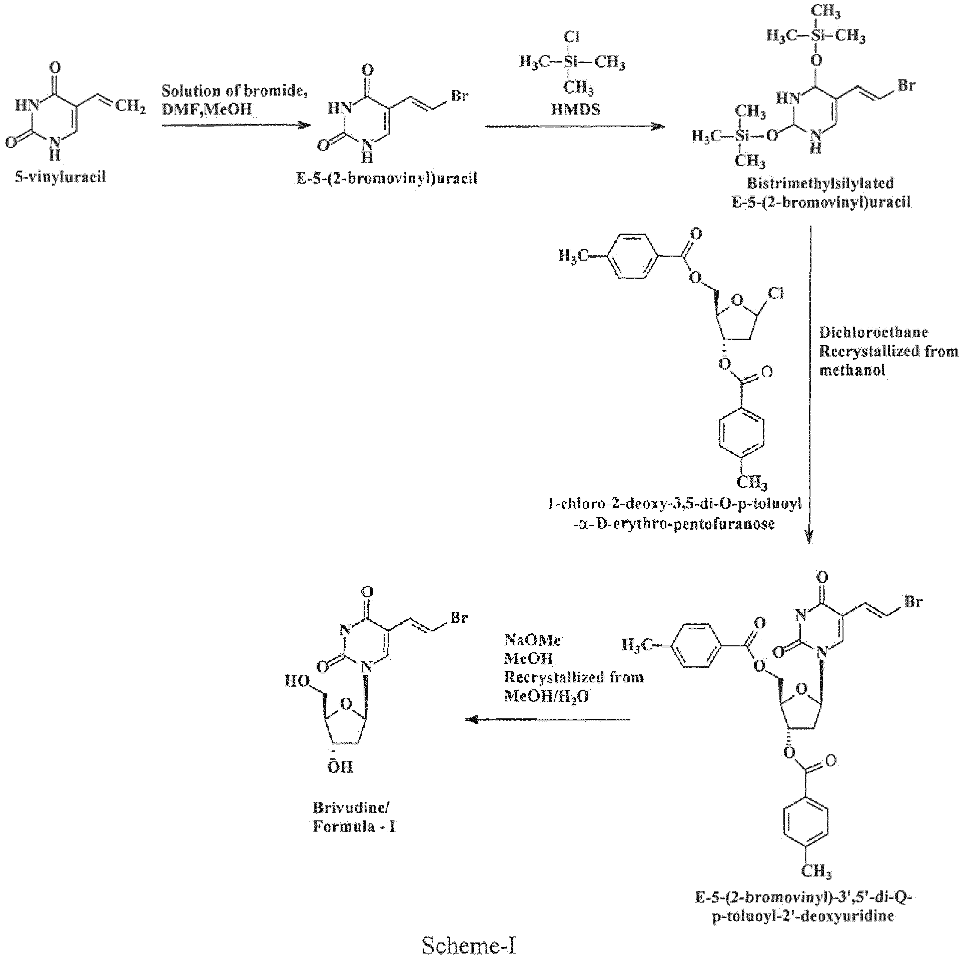

The major disadvantages associated with the process disclosed in DE 2915254 includes the use of expensive starting material, formation of unwanted excess of α-isomer. The undesired α-isomer result in a final product of low purity, making chromatographic purification methods not feasible at an industrial scale. Additionally, the process involves the use of bromine for the synthesis of E-5-(2-bromovinyl)uracil, which is a well known carcinogen.

GB 2125399 describe another process for the preparation of Brivudine involves the bromination and simultaneous dehydrohalogenation of 5-ethyl-2'-deoxyuridine in the presence of halogenated hydrocarbon solvent. The process is depicted in the below as Scheme - II:

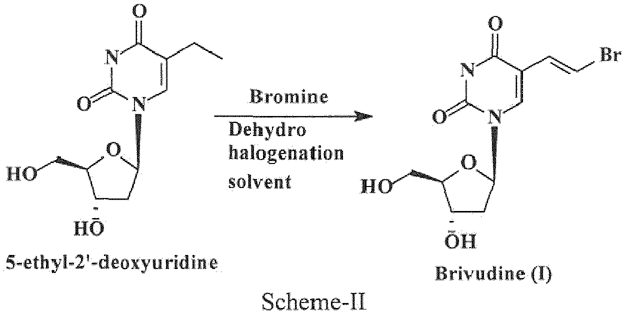

The major disadvantages associated with the process disclosed in GB 2125399 includes the use of bromine for bromination, which make the process carcinogenic and the use of halogenated solvents like chloroform, carbon tetrachloride and dichloroethane for bromination makes the process environmentally hazardous.

US 2010298530 A1 discloses a process for the preparation of Brivudine by coupling 5-Iodo deoxyuridine with methyl acrylate in presence of palladium acetate to form (E)-5-(carbomethoxyvinyl)-2'-deoxyuridine, which is hydrolyzed with sodium hydroxide solution to obtain (E)-5-(carboxyvinyl)-2'-deoxyuridine, which undergoes bromination by using N-Bromo succinimide [NBS] to obtain Brivudine. The process is depicted in the below as Scheme - III:

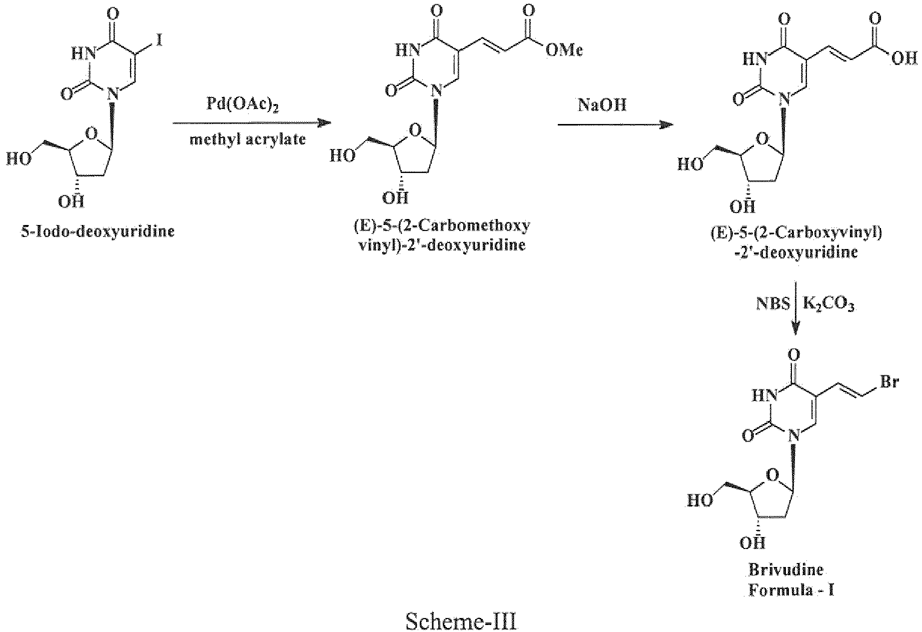

The major disadvantages associated with the process disclosed in US 2010298530 A1 includes the use of expensive palladium acetate as catalyst and chromatographic purification method not feasible at an industrial scale. In addition, the above process involves the use of methyl acrylate and the process liberates iodine, which are highly carcinogenic. It makes the process environment unfriendly.

The inventors of the present invention found an alternative route to prepare Brivudine (I), which is industrial feasible, can avoid the use of potentially hazardous, expensive chemicals and to minimize the formation of undesired α-isomer and the other process related impurities. The present invention directed towards a process for the preparation of Brivudine of Formula - I with high purity and high yield.

### OBJECTIVE OF THE INVENTION

The objective of the present invention is to provide a novel compound 2'-deoxyuridine of Formula-II and its conversion to Brivudine of Formula-I or pharmaceutically acceptable salts thereof. 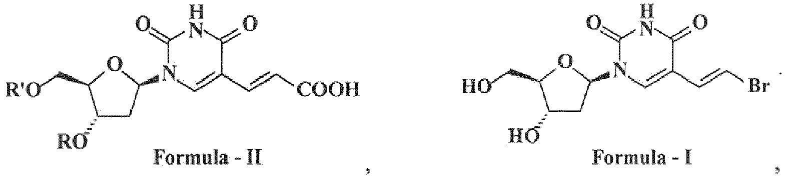 wherein, R and R' are same or different selected from hydroxy protecting groups.

Another objective of the present invention is a novel compound 2'-deoxyuridine of Formula - II. 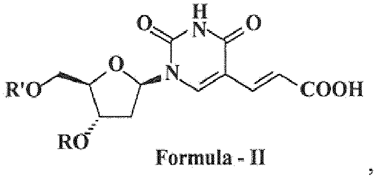 wherein, R and R' are same or different selected from hydroxy protecting groups.

### SUMMARY OF THE INVENTION

In an embodiment, the present invention provides a process for the preparation of brivudine of Formula - I, 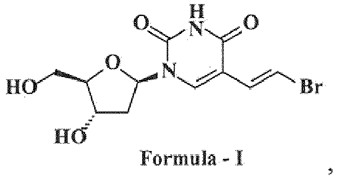 which comprises:
(i) reacting uracil acrylic acid of Formula -IV with a compound of formula - VI to obtain uridine acrylic acid of Formula-II; 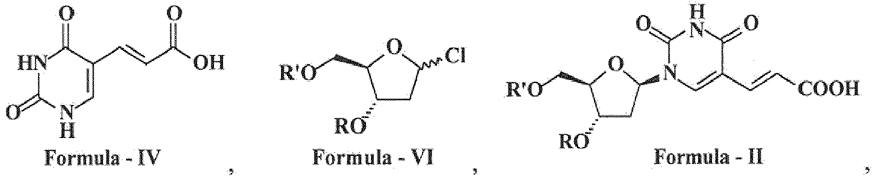 wherein, R and R' are same or different selected from hydroxy protecting groups;
(ii) brominating the uridine acrylic acid of Formula-II to obtain a compound of Formula-III; 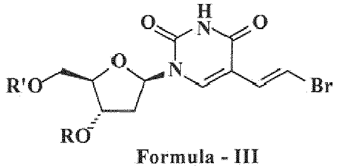
(iii) deprotecting the hydroxy group of the compound of Formula-III to obtain Brivudine of Formula - I.

In another embodiment, the present invention provides a compound of Formula - II, 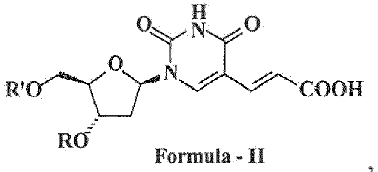 wherein, R and R' are same or different selected from hydroxy protecting groups.

### DETAILED DESCRIPTION OF THE INVENTION

- I. The present invention relates to a process for the preparation of Brivudine of Formula

The process for preparation of Brivudine of Formula - I comprises, reacting uracil acrylic acid of Formula-IV with a compound of formula-VI to obtain uridine acrylic acid of Formula-II, brominating uridine acrylic acid of Formula-II to obtain compound of Formula - III, deprotecting the hydroxy group of the compound of Formula - III to obtain Brivudine of Formula - I.

The term "hydroxy protecting group," defined as R and R', as used herein refers to any chemical group introduced into a molecule by chemical modification of a functional group in a subsequent chemical reaction. Non-limiting examples of hydroxy protecting groups comprise acetyl, benzoyl, substituted benzoyl, benzyl, β-methoxyethoxymethyl ether, dimethoxytrityl, methoxymethyl ether, methoxytrityl, p-methoxybenzyl ether, pivaloyl, tetrahydropyranyl, trityl, and trimethylsilyl.

Reaction of uracil acrylic acid of Formula - IV with a compound of formula - VI is carried out in the presence of a silylating agent.

The silyating agent is selected from Hexamethyl disilazane, trimethyl chlorosilane or mixture thereof.

The bromination is carried out by using a brominating agent selected from Bromine, N-bromosuccinimide [NBS], hydrogen bromide [HBr], sodium bromide, cyanogen bromide [BrCN], 1,3-dibromo-5,5-dimethyl hydantoin [DBH], 2,4,4,6-tetrabromo-2,5-cyclohexadien-1-one [TBCD] or mixtures thereof.

The reaction is carried out at a temperature from 0 °C to 40 °C, preferably at 20 °C to 30 °C.

The bromination is carried out in the presence of a base and solvent.

The deprotection is carried out in the presence of a base and solvent.

Base used in above reaction system comprises organic base selected from lithium methoxide, sodium methoxide, potassium methoxide; tetrabutyl ammonium methoxide, lithium isopropoxide, triethyl amine, diisopropyl methyl amine, methyl amine, dimethyl amine or mixtures thereof and inorganic base selected from alkaline or alkaline earth metal hydroxide, alkaline or alkaline earth metal carbonate or mixtures thereof.

Solvent used in above reaction system comprises water, alcohols, halogenated hydrocarbons, hydrocarbons, amides, sulfoxides, nitriles, esters, ethers, ketones and mixtures thereof. The alcohols comprises C₁₋₆ alcohols selected from methanol, ethanol, butanol, isopropanol and the like; halogenated hydrocarbons selected from methylene chloride, ethylene chloride, chloroform and the like; hydrocarbons selected from hexane, cyclohexane, toluene, xylene and the like; amides selected from dimethyl formamide, dimethyl acetamide, N-methyl pyrrolidinone and the like; sulfoxides selected from but are not limited to dimethyl sulfoxide and the like; nitriles selected from acetonitrile, propionitrile and the like; esters comprises, ethyl acetate and butyl acetate and the like; ethers selected from diethyl ether, diisopropyl ether, t-butyl methyl ether, 1,4-dioxane, tetrahydrofuran and the like; ketones selected from acetone, methyl ethyl ketone, methyl isopropyl ketone and the like and mixtures thereof.

The following example(s) illustrate the nature of the invention and are provided for illustrative purposes only and should not be construed to limit the scope of the invention.

### EXAMPLE-1:

### PREPARATION OF URIDINE ACRYLIC ACID

Trimethylchlorosilane (0.6 ml, 5 mmol) was added to the suspension of uracil acrylic acid (6.35g, 34.9 mmol) in hexamethyldisilazane (70 ml) and resulting mixture was refluxed till the clear solution was obtained. Hexamethyldisilazane was evaporated under vacuum and further co-evaporated with o-xylene to remove the traces of hexamethyldisilazane to yield viscous oily silylated uracil acrylic acid. The silylated uracil acrylic acid was dissolved in dichloromethane (100 ml) under nitrogen atmosphere, cooled at 0-10 °C. Anhydrous zinc chloride (0.63 grams, 4.6 mmol) and chloro-sugar (10 grams, 23.2 mmol) were added to the above solution. The reaction was monitored by qualitative HPLC and was essentially completed in 3 hours. After completion of the reaction dichloromethane was evaporated under vacuum. Methyl tert-butyl ether (100 ml) was added and stirred for 1 hour at 40-45 °C. The product was isolated after filtration at 25-30 °C. HPLC analysis showed the complete consumption of chloro-sugar and the ratio β/α = 98.
Yield: 75%

### EXAMPLE-2:

### PREPARATION OF DIBENZOYL BRIVUDINE

Uridine acrylic acid (5.0 grams, 8.69 mmol) was suspended in a mixture of tetrahydrofuran (45.0 ml) and water (5.0 ml). Potassium acetate (0.93 grams, 9.56 mmol) and N-bromosuccinamide (1.70 grams, 9.56 mmol) was added to the suspension and the resulting mixture was stirred for 2 hours at 25-30 °C. The solvent was removed under reduced pressure to the dryness and methanol (50 ml) was poured, suspension was stirred for 1 hour at 25-30 °C. The product was isolated after filtration.
Yield: 55%

### EXAMPLE-3:

### PREPARATION OF BRIVUDINE (FORMULA - I)

Dibenzoyl Brivudine (6 grams, 9.83 mmol) was suspended in methanol (30 ml) at 20-30 °C. A solution of 25 % w/w sodium methoxide (2.76 grams, 12.78 mmol) in methanol was added to the suspension and was allowed for 1hour at the same temp. The reaction was monitored by qualitative HPLC. Methanol was evaporated under vacuum and resulting residue was dissolved in water (25 ml). The aqueous mass was washed with methylene dichloride (2x20 ml) and the product was isolated from water at pH 2-3.
Yield: 85%

## Claims

1. A process for the preparation of Brivudine of Formula - I, 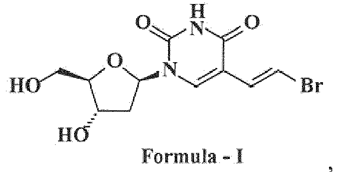 which comprises the steps of:
a) reacting uracil acrylic acid of Formula - IV with a compound of formula - VI to obtain uridine acrylic acid of Formula - II; 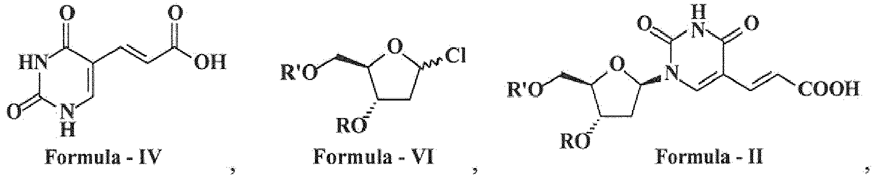 wherein, R and R' are the same or different and are selected from hydroxy protecting groups;
b) brominating the uridine acrylic acid of Formula - II to obtain a compound of Formula-III; 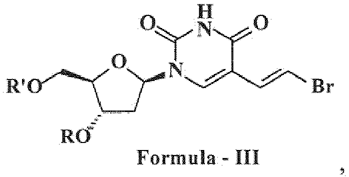
c) deprotecting the hydroxy group of the compound of Formula - III to obtain Brivudine of Formula-I .

2. The process according to claim 1, wherein step (a) is carried out in the presence of a silylating agent.

3. The process according to claims 1 and 2, wherein the silylating agent is selected from Hexamethyl disilazane, trimethyl chlorosilane or a mixture thereof.

4. The process according to claims 1-3, wherein the brominating agent in step (b) is selected from Bromine, N-bromosuccinimide, hydrogen bromide, sodium bromide, cyanogen bromide, 1,3-dibromo-5,5-dimethyl hydantoin, 2,4,4,6-tetrabromo-2,5-cyclohexadien-1-one or mixtures thereof.

5. The process according to claims 1-4, wherein the deprotection of step (c) is carried out in the presence of a base and a solvent.

6. The process according to claims 1-5, wherein the base used in step (c) is selected from an organic base such as lithium methoxide, sodium methoxide, potassium methoxide; tetrabutyl ammonium methoxide, lithium isopropoxide, triethyl amine, diisopropyl methyl amine, methyl amine, dimethyl amine or mixtures thereof and an inorganic base such as alkaline or alkaline earth metal hydroxide, alkaline or alkaline earth metal carbonate or mixtures thereof.

7. The process according to claims 1-6, wherein the solvent is selected from water, alcohols, halogenated hydrocarbons, hydrocarbons, amides, sulfoxides, nitriles, esters, ethers, ketones and mixtures thereof.

8. A novel intermediate of Brivudine of Formula - II,
